# EUROPEAN PATENT APPLICATION

(11) **EP 4 450 499 A1**
(43) Date of publication of application: **23.10.2024**
(21) Application number: 22906521.4
(22) Date of filing: 12.12.2022
(51) Int. Cl.: C07D 471/04, A61K 31/519, A61P 35/00

(54) **FGFR4 INHIBITOR ACID SALT, PREPARATION METHOD THEREFOR, AND USE THEREOF**

(30) Priority: 13.12.2021 CN 202111522461
(71) Applicant: Abbisko Therapeutics Co., Ltd., Shanghai 201203 (CN)
(72) Inventor: ZHANG, Lei, Shanghai 201203 (CN); HOU, Qiwen, Shanghai 201203 (CN); YU, Hongping, Shanghai 201203 (CN)
(74) Representative: dompatent von Kreisler Selting Werner - Partnerschaft von Patent- und Rechtsanwälten mbB
(86) International application number: PCT/CN2022/138455
(87) International publication number: WO 2023/109776

(57) **Abstract**

The present invention relates to an FGFR4 inhibitor acid salt, a preparation method therefor, and a use thereof. The FGFR4 inhibitor is a compound *N*-((35,45)-3-((6-(2,6-difluoro-3,5-dimethoxyphenyl)-8-(3-methoxy-3-methylazetidin-1-yl)pyrido[3,4-*d*]pyrimidin-2-yl)amino)tetrahydro-2*H*-pyran-4-yl)acrylamide having the structure represented by formula (I), and the acid salt is *p*-toluenesulfonate. The physical and chemical properties such as the solubility and bioavailability of the compound represented by formula (I) in the free form are greatly increased, and the development requirements of clinical medicine preparations can be met. The present invention has vital clinical application value for p-toluenesulfonate and has the prospect of developing into a new generation of FGFR4 small-molecule inhibitors.

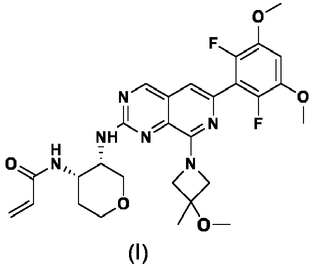

## Description

### TECHNICAL FIELD

The present invention belongs to the field of drug development, and particularly relates to an FGFR4 inhibitor acid salt, preparation method therefor and use thereof.

### BACKGROUND

Fibroblast growth factors (FGFs) are a family of 22 structurally related polypeptides with distinct biological activities, and the corresponding receptors of FGFs (FGFRs) belong to a family of receptor tyrosine kinases, RPTK. Four receptors, i.e., FGFR1, FGFR2, FGFR3 and FGFR4, have been discovered, and their interaction with the corresponding ligands FGF leads to receptor dimerization and autophosphorylation, thereby initiating multiple downstream signaling cascades including MAPK and AKT. Hepatocellular carcinoma (HCC) is one of the leading causes of cancer-related deaths in China and is one of the cancers having the fastestgrowing incidence every year. The current first-line treatment regimen is sorafenib, and there is no approved second-line drug. There is still a need for targeted drugs with anti-tumor agents. 5-10% of hepatocellular carcinoma patients exhibit overexpression of FGF19, while FGFR4 is the predominant FGFR present in human hepatocytes, and its high expression in hepatocytes is considered to be associated with the aggressiveness of hepatocellular carcinoma. Therefore, FGFR4 plays a very important role in liver cancer. In addition, the interaction of FGF19 and FGFR4 is also thought to be associated with the aggressiveness of other cancer types (e.g., gastric cancer, prostate cancer, lung cancer, colorectal cancer, pancreatic cancer and ovarian cancer).

At present, the high-selectivity FGFR4 inhibitor can effectively treat cancer caused by abnormal FGFR4 signaling, and can avoid the related side effects of hyperphosphatemia and the like caused by FGFR1-3 inhibition. The highly selective small-molecule inhibitor of FGFR4 has great application prospects in the field of target and immune therapy for tumors, and the FGFR4 inhibitor, as a good drug candidate, can meet the requirements of targeted drugs for liver cancer and other tumors at home and abroad and has the advantages of good safety and stronger specificity.

During the process of long-term research, Abbisko Therapeutics Co., Ltd. found a small-molecule compound of a novel structure having the effect of highly selective inhibition of FGFR4 (related patent application: WO2018113584A1; international publication date: Jun. 28, 2018), a representative compound of which is shown below:

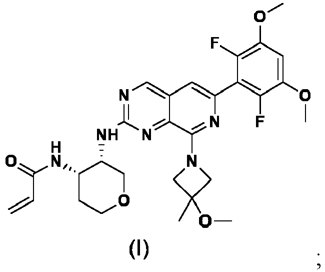

The compound name is *N*-((3*S*,4*S*)-3-((6-(2,6-difluoro-3,5-dimethoxyphenyl)-8-(3-methoxy-3-methylazetidin-1-yl)pyrido[3,4-*d*]pyrimidin-2-yl)amino)tetrahydro-2*H*-pyran-4-yl)acrylamide (compound of formula (I)). The compound can remarkably improve the inhibition effect of FGFR4 target and the selectivity over other FGFR1-3 kinase receptors, and meet the requirements of target and immune therapy for liver cancer, prostate cancer, pancreatic cancer, esophageal cancer, gastric cancer, lung cancer, breast cancer, ovarian cancer, colon cancer, skin cancer, glioblastoma, rhabdomyosarcoma and the like at present stage at home and abroad.

However, no raw materials and processes suitable for clinical and industrial applications were developed at the time of filing the patent application, and no further study was made as to whether the compound of formula (I) is suitable for drug development. The inventor discovers in later research that the free base compound disclosed in WO2018113584A1 is difficult to dissolve in water and physiological vehicles, has poor bioavailability, can not meet clinical requirements and is not suitable for clinical formulation development. Therefore, to meet the needs of clinical research and the launch of drug formulations, there is an urgent need to further study the aggregation state of the drug to improve the physicochemical property of the compound and thereby meet the needs of pharmacy or clinical application and develop a salt form or crystalline form of the compound suitable for drug development to overcome the defects in the prior art.

### SUMMARY

To solve the problems in the prior art, the inventor further studied the aggregation state of the compound of formula (I) *N*-((3*S*,4*S*)-3-((6-(2,6-difluoro-3,5-dimethoxyphenyl)-8-(3-methoxy-3-methylazetidin-1-yl)pyrido[3,4-*d*]pyrimidin-2-yl)amino)tetrahydro-2*H*-pyran-4-yl)acrylamide, and in long-term research, the inventor surprisingly found that the *p-*toluenesulfonate salt can greatly improve the solubility and bioavailability of the compound of formula (I) in free form, and the physicochemical properties such as hygroscopicity and chemical stability can also meet the requirements for industrial production, and therefore the *p-*toluenesulfonate salt of the compound can satisfy the needs of clinical drug formulation development. The *p*-toluenesulfonate salt of the present invention can be widely used in the preparation of medicaments for treating cancers, particularly liver cancer, prostate cancer, pancreatic cancer, esophageal cancer, gastric cancer, lung cancer, breast cancer, ovarian cancer, colon cancer, skin cancer, glioblastoma or rhabdomyosarcoma, has vital clinical application value and is expected to be developed in an accelerated way into a new generation of FGFR4 small-molecule inhibitors.

The first aspect of the present invention provides a *p*-toluenesulfonate salt of a compound of formula (I):

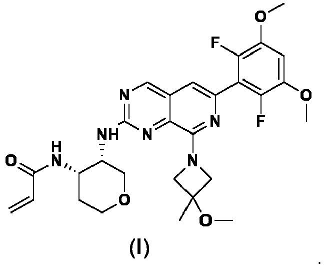

As a preferred embodiment, the *p*-toluenesulfonate salt of the compound of formula (I) is a crystalline compound.

As a further preferred embodiment, an X-ray powder diffraction pattern (XRPD) of the *p-*toluenesulfonate salt of the compound of formula (I) comprises peaks at angles of diffraction (2θ) of 9.02 ± 0.2°, 17.18 ± 0.2°, 17.68 ± 0.2°, 19.60 ± 0.2° and 22.90 ± 0.2°.

As a more further preferred embodiment, an X-ray powder diffraction pattern (XRPD) of the *p*-toluenesulfonate salt of the compound of formula (I) comprises peaks at angles of diffraction (2θ) of 9.02 ± 0.2°, 17.18 ± 0.2°, 17.68 ± 0.2°, 19.60 ± 0.2°, 22.90 ± 0.2°, 10.60 ± 0.2°, 12.72 ± 0.2°, 18.56 ± 0.2°, 21.22 ± 0.2° and 24.90 ± 0.2°.

As a more further preferred embodiment, an X-ray powder diffraction pattern (XRPD) of the *p*-toluenesulfonate salt of the compound of formula (I) comprises peaks at angles of diffraction (2θ) of 9.02 ± 0.2°, 17.18 ± 0.2°, 17.68 ± 0.2°, 19.60 ± 0.2°, 22.90 ± 0.2°, 10.60 ± 0.2°, 12.72 ± 0.2°, 18.56 ± 0.2°, 21.22 ± 0.2°, 24.90 ± 0.2°, 15.32 ± 0.2°, 23.82 ± 0.2°, 24.12 ± 0.2°, 24.60 ± 0.2° and 25.58 ± 0.2°.

As a preferred embodiment, an X-ray powder diffraction pattern of the *p*-toluenesulfonate salt of the compound of formula (I) comprises peaks at angles of diffraction (2θ) of 9.02 ± 0.2°, 10.60 ± 0.2°, 12.04 ± 0.2°, 12.72 ± 0.2°, 15.32 ± 0.2°, 17.18 ± 0.2°, 17.68 ± 0.2°, 18.56 ± 0.2°, 19.60 ± 0.2°, 20.60 ± 0.2°, 21.22 ± 0.2°, 22.90 ± 0.2°, 23.82 ± 0.2°, 24.12 ± 0.2°, 24.60 ± 0.2°, 24.90 ± 0.2°, 25.58 ± 0.2°, 27.78 ± 0.2°, 29.04 ± 0.2°, 31.48 ± 0.2°, 36.60 ± 0.2° and 38.16 ± 0.2°.

As the most preferred embodiment, an X-ray powder diffraction pattern of the *p-*toluenesulfonate salt of the compound of formula (I) comprises peaks that are substantially identical to the peaks at angles of diffraction (2θ) shown in Table 1, and the angles of diffraction (2θ) and the related intensity data (± 0.2°) are as follows:

| 2θ(°) | Intensity % | 2θ(°) | Intensity % |
|---|---|---|---|
| 9.02 | 40.9 | 27.78 | 6.8 |
| 10.60 | 19.6 | 29.04 | 8.4 |
| 12.04 | 7.5 | 29.51 | 1.7 |
| 12.72 | 29.8 | 30.62 | 2.8 |
| 14.88 | 4.3 | 31.48 | 8.1 |
| 15.32 | 12.4 | 31.92 | 2.4 |
| 17.18 | 47 | 32.38 | 1.2 |
| 17.68 | 100 | 32.84 | 3.7 |
| 18.56 | 23.2 | 34.04 | 2.4 |
| 19.60 | 61.3 | 35.10 | 2.2 |
| 20.60 | 8.1 | 36.60 | 6.9 |
| 21.22 | 29.1 | 37.10 | 3 |
| 22.90 | 84.9 | 38.16 | 5.1 |
| 23.82 | 18.8 | 39.72 | 3.3 |
| 24.12 | 17.7 | 40.14 | 2.4 |
| 24.60 | 14.5 | 41.06 | 2.1 |
| 24.90 | 31.5 | 42.38 | 3.5 |
| 25.58 | 13.8 | 42.72 | 2 |
| 26.20 | 4.2 | 44.24 | 1.9 |
| 26.66 | 2.9 | | |

As a preferred embodiment, the unit cell of the *p*-toluenesulfonate salt of the compound of formula (I) is P1, a = 9.079 (3) Å, b = 10.561 (3) Å, c = 10.882 (3) Å, and the unit cell volume is 908.1 (4) Å³.

As a preferred embodiment, the *p*-toluenesulfonate salt of the compound of formula (I) is a hydrate.

As a more preferred embodiment, the *p*-toluenesulfonate salt hydrate of the compound of formula (I) comprises 1-3 water molecules per molecule.

As a more further preferred embodiment, the *p*-toluenesulfonate salt hydrate of the compound of formula (I) comprises 1, 2 or 3 water molecules per molecule.

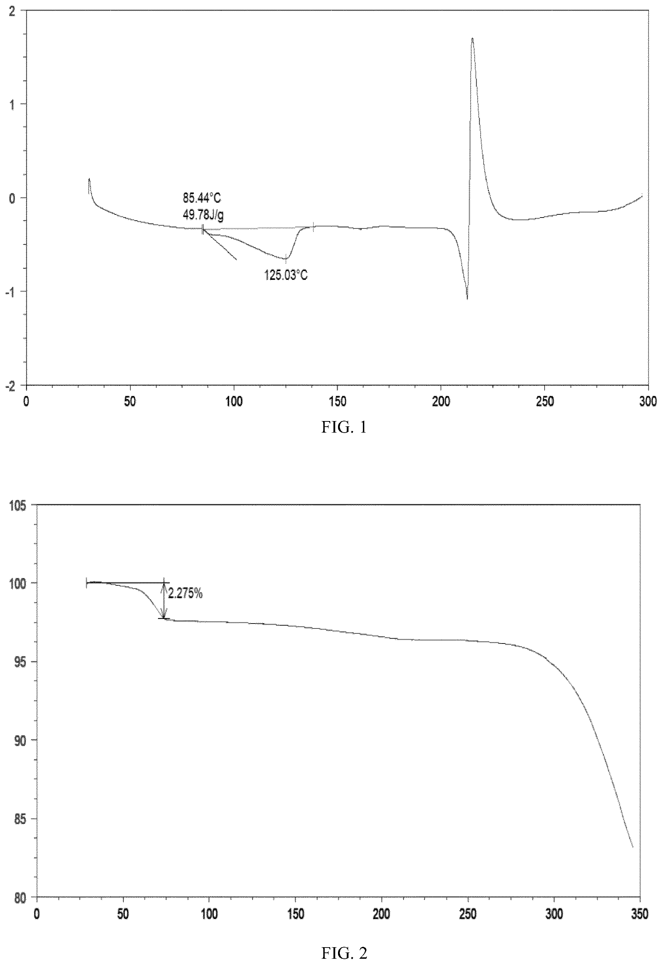

As the invention of the present application, the applicant designates the crystalline *p-*toluenesulfonate salt of the compound of formula (I) having any one of the X-ray powder diffraction data as a crystalline form A (Form A), and the obtained crystal is physically characterized by DSC, TGA and XRPD and shown as a relatively good crystalline solid, and the DSC and TGA results thereof indicate that the acidic salt of the crystalline compound of formula (I) is a monohydrate, as shown in FIG. 1 and FIG. 2.

The second aspect of the present invention provides a preparation method of the *p-*toluenesulfonate salt of the compound of formula (I) described above, which comprises the following steps:
1) dissolving or dispersing the compound of formula (I) in free form in an aqueous solvent or a suitable organic solvent, and adding *p*-toluenesulfonic acid or a *p*-toluenesulfonic acid solution to the above system for a salt forming reaction; or adding the compound of formula (I) in free form to a *p*-toluenesulfonic acid solution for a salt forming reaction;
2) collecting a *p*-toluenesulfonate salt of the compound of formula (I) in the form of a solid product precipitated during the salt forming reaction; or creating a degree of supersaturation of the salt forming system to obtain a *p*-toluenesulfonate salt of the compound of formula (I) in the form of a solid product.

As a preferred embodiment, a method for creating the degree of supersaturation of the salt forming system in step 2) includes one or more of: adding a seed crystal, volatilizing a solvent, adding an anti-solvent and cooling.

As a preferred embodiment, the obtained *p*-toluenesulfonate salt of the compound of formula (I) in the form of a solid product is a crystalline *p*-toluenesulfonate salt of the compound of formula (I).

As a preferred embodiment, the suitable organic solvent used in the salt forming process of step 1) is selected from the group consisting of alcohols, chloroalkanes, ketones, ethers, cyclic ethers, esters, alkanes, cycloalkanes, benzenes, amides, sulfoxides and mixtures thereof.

As a preferred embodiment, the suitable organic solvent is selected from the group consisting of methanol, ethanol, *n*-propanol, isopropanol, dichloromethane, acetonitrile, acetone, 1,4-dioxane, tetrahydrofuran, *N*,*N*-dimethylformamide, ethyl acetate, isopropyl acetate, methyl *tert*-butyl ether, 2-methoxyethyl ether and mixtures thereof.

The third aspect of the present invention provides a pharmaceutical composition comprising a therapeutically effective dose of the *p*-toluenesulfonate salt of the compound of formula (I) described above and a pharmaceutically acceptable carrier.

The fourth aspect of the present invention provides a use of the *p*-toluenesulfonate salt of the compound of formula (I) in the preparation of a medicament as an FGFR4 inhibitor.

The fifth aspect of the present invention provides a use of the *p*-toluenesulfonate salt of the compound of formula (I) in the preparation of a medicament for treating liver cancer, prostate cancer, pancreatic cancer, esophageal cancer, gastric cancer, lung cancer, breast cancer, ovarian cancer, colon cancer, skin cancer, glioblastoma or rhabdomyosarcoma.

The sixth aspect of the present invention provides a method for treating liver cancer, prostate cancer, pancreatic cancer, esophageal cancer, gastric cancer, lung cancer, breast cancer, ovarian cancer, colon cancer, skin cancer, glioblastoma or rhabdomyosarcoma, which comprises administering to a patient in need thereof a therapeutically effective amount of the *p-*toluenesulfonate salt of the compound of formula (I) described above or the pharmaceutical composition described above.

### BRIEF DESCRIPTION OF THE DRAWINGS

FIG. 1 is a differential scanning calorimetry graph of the *p*-toluenesulfonate salt of the compound of formula (I) (Form A); the X-axis is temperature (°C), and the Y-axis is heat flow (W/G).
FIG. 2 is a thermogravimetric analysis graph of the *p*-toluenesulfonate salt of the compound of formula (I) (Form A); the X-axis is temperature (°C), and the Y-axis is percent weight loss (%).
FIG. 3 is an X-ray powder diffraction pattern of the *p*-toluenesulfonate salt of the compound of formula (I) (Form A); the X-axis is the value of angle 2Θ of diffraction peaks (°), and the Y-axis is the intensity of the peak.
FIG. 4 is a simulated powder diffraction pattern of the *p*-toluenesulfonate salt of the compound of formula (I) (Form A) obtained by single crystal X-ray diffraction (lower) and an observed XRPD pattern of the *p*-toluenesulfonate salt of the compound of formula (I) (Form A) (upper). The abscissa represents the value of 2θ (°), and the ordinate represents peak intensity.
FIG. 5 is a unit cell diagram of a single crystal of the *p*-toluenesulfonate salt of the compound of formula (I) (Form A).
FIG. 6 is a DVS graph of the *p*-toluenesulfonate salt of the compound of formula (I) (Form A). The abscissa represents relative humidity (%), and the ordinate represents weight change (%).
FIG. 7 is a time/solubility curve of the *p*-toluenesulfonate salt of the compound of formula (I) (Form A) and the compound of formula (I) in free form in a vehicle of 25% PEG300 / 5% Solutol HS15 / 70% water. The abscissa is time (hr), and the ordinate is concentration (mg/mL).

### DETAILED DESCRIPTION

Because of different crystalline forms, the drugs may have different bioavailability, solubility, melting point, chemical and physical stability, etc., thereby affecting the safety and effectiveness of the drugs. To develop salt forms or crystalline forms suitable for drug development, the inventor carried out intensive research on the compound of formula (I), *N-*((3*S*,4*S*)-3-((6-(2,6-difluoro-3,5-dimethoxyphenyl)-8-(3-methoxy-3-methylazetidin-1-yl)pyrido[3,4-*d*]pyrimidin-2-yl)amino)tetrahydro-2*H*-pyran-4-yl)acrylamide, screened a large number of salt forms and crystalline forms of the compound of formula (I), and finally found that the *p*-toluenesulfonate salt can greatly improve the solubility and bioavailability of the compound of formula (I) in free form, and the physicochemical properties such as hygroscopicity and chemical stability can also meet the requirements for industrial production, and therefore the p-toluenesulfonate salt of the compound can satisfy the needs of clinical drug formulation development and is expected to be developed in an accelerated way into a new generation of FGFR4 small-molecule inhibitors.

Detailed description: unless otherwise stated, the following terms used in the specification and claims have the following meanings.

"Pharmaceutical composition" refers to a mixture containing one or more of the compounds described herein or a physiologically/pharmaceutically acceptable salt or pro-drug thereof, and other chemical components, for example, physiologically/pharmaceutically acceptable carriers and excipients. The purpose of the pharmaceutical composition is to promote the administration to an organism, which facilitates the absorption of the active ingredient, thereby exerting biological activities.

"Polymorph" refers to crystalline forms having the same chemical composition but different spatial arrangements of the molecules, atoms and/or ions making up the crystal. While polymorphs have the same chemical composition, they differ in packing and geometrical arrangement, and may exhibit different physical properties such as melting point, shape, color, density, hardness, deformability, stability, solubility, dissolution rate, and the like. Depending on their temperature-stability relationship, two polymorphs may be either monotropic or enantiotropic. For a monotropic system, the relative stability between the two solid phases remains unchanged as the temperature is changed. In contrast, in an enantiotropic system, there exists a transition temperature at which the stabilities of the two phases reverse. The phenomenon that a compound exists in different crystalline structures is called drug polymorphism.

The various crystalline structures of the present invention can be distinguished from one another using various analytical techniques known to those of ordinary skill in the art. Such techniques include, but are not limited to, X-ray powder diffraction (XRPD), differential scanning calorimetry (DSC) and/or thermogravimetric analysis (TGA).

### Methods and Materials

### 1.1 X-ray powder diffraction

Those of ordinary skill in the art will recognize that X-ray powder diffraction patterns may be obtained with measurement errors that depend on the measurement conditions used. The intensity in the X-ray powder diffraction pattern may fluctuate depending on the materials used. The relative intensity may also vary with experimental conditions and accordingly, the exact intensity should not be taken into account. In addition, the measurement error of the angle of diffraction in the conventional X-ray powder diffraction is generally about 5% or less, and such a degree of measurement error should be regarded as belonging to the above-mentioned angle of diffraction. Therefore, the crystalline structures of the present invention are not limited to crystalline structures that provide X-ray powder diffraction patterns that are identical to X-ray powder diffraction patterns depicted in the drawings disclosed herein. Any crystalline structure having an X-ray powder diffraction pattern substantially the same as those disclosed in the drawings falls within the scope of the present invention. Those skilled in the art would have the ability to determine substantially the same X-ray powder diffraction patterns. Calibration is performed with other suitable standards known to those skilled in the art. However, the relative intensity may vary with crystal size and shape.

The crystalline forms of the compounds of the present invention were characterized by their X-ray powder diffraction patterns. Thus, the X-ray powder diffraction patterns of the salts were collected on a Bruker D8 Discover X-ray powder diffractometer with GADDS (general area diffraction detector system) CS operating in reflection mode using Cu Kα radiation (1.54 Å). The tube voltage and amperage were set to 40 kV and 40 mA, respectively, for acquisition scans. The samples were scanned for a period of 60 seconds covering a range of 3.0° to 40° in 2Θ. The diffractometer was calibrated for peak positions in 2Θ using a corundum standard. All analyses were usually conducted at 20-30 °C. Data were collected and integrated using GADDS for WNT software version 4.1.14T. Diffraction patterns were analyzed using DiffracPlus software with Eva version 9.0.0.2 released in 2003.

XRPD samples were prepared as follows: usually a sample was placed on a monocrystalline silicon wafer, and the sample powder was pressed with a glass slide or an equivalent to ensure that the sample has a flat surface and a proper height. The sample holder was then placed in the Bruker XRPD instrument and an X-ray powder diffraction pattern was collected using the instrument parameters described above. Measurement differences associated with such X-ray powder diffraction analyses result from a variety of factors including: (a) errors in sample preparation (e.g., sample height), (b) instrument errors, (c) calibration errors, (d) operator errors (including those errors present when determining peak positions), and (e) the nature of the material (e.g., preferred orientation errors). Calibration errors and sample height errors often result in a shift of all the peaks in the same direction. In general, this calibration factor will bring the measured peak positions into an agreement with the expected peak positions and may be in the range of the expected 2Θ value ± 0.2°.

### 1.2 Differential scanning calorimetry (DSC)

Differential scanning calorimetry (DSC) experiments were carried out in a TA instruments^{™} model Q2000. The sample (about 1-6 mg) was weighed in an aluminum pan and recorded to exactly a hundred of a milligram and transferred to the DSC. The instrument was purged with nitrogen at 50 mL/min. Data were collected between room temperature and 350 °C at a heating rate of 10 °C/min. The plot was made with the endothermic peaks pointing down. However, those skilled in the art will note that in DSC measurement, there is a certain degree of variability in actual measured start and peak temperatures, depending on heating rate, crystal shape and purity, and other measurement parameters.

### 1.3 Thermogravimetric analysis (TGA)

Thermogravimetric analysis (TGA) experiments were carried out in a TA instruments^{™} model Q500. The sample (about 10-30 mg) was placed in a platinum pan previously tared. The weight of the sample was measured accurately and recorded to a thousand of a milligram by the instrument. The furnace was purged with nitrogen at 100 mL/min. Data were collected between room temperature and 300 °C at a heating rate of 10 °C/min.

### 1.4 Dynamic vapor sorption (DVS)

The experimental method of characterizing the crystalline acidic salts of the compound of formula (I) using dynamic vapor sorption (DVS) is as follows: a small amount of crystalline acidic salt powder of the compound of formula (I) was placed in a precision sample pan that came with the instrument; after the sample was loaded, the aluminum pan was sent into the instrument for analysis. In the present patent, the instruments used for dynamic vapor sorption were all the DVS Intrinsic model, and the experimental parameters were set as follows: nitrogen was set as carrier gas, the constant temperature was set to 25 °C, the rate of mass percentage change over a unit of time (dm/dt) = 0.01%/min was used as a criterion for determining if an equilibrium is reached, and the program humidity change cycle was set as follows: the initial relative humidity was 0%, the relative humidity was 90% at the end of the cycle, 2 cycles were set, and each 10% R.H. change was a step.

Reagents in examples of the present invention are known and commercially available, either commercially available industrial grade or analytical grade reagents. Or the regents may be synthesized using or following methods known in the art, and the API starting materials are prepared according to patent WO2018113584A1.

Unless otherwise stated, all reactions of the present invention are carried out under a dry nitrogen or argon atmosphere with continuous magnetic stirring, the solvent is a dry solvent, and the temperature is in degree centigrade (°C). The term "room temperature" or "RT" used herein refers to an ambient temperature of 20-25 °C (68-77 F).

The present invention is further explained in detail through the following examples, which are intended to illustrate specific embodiments of the present invention only and should not be construed as limiting the scope of the present invention in any way.

### Preparation of Specific Examples

### Example 1. Preparation of p-toluenesulfonate Salt of Compound of Formula (I)

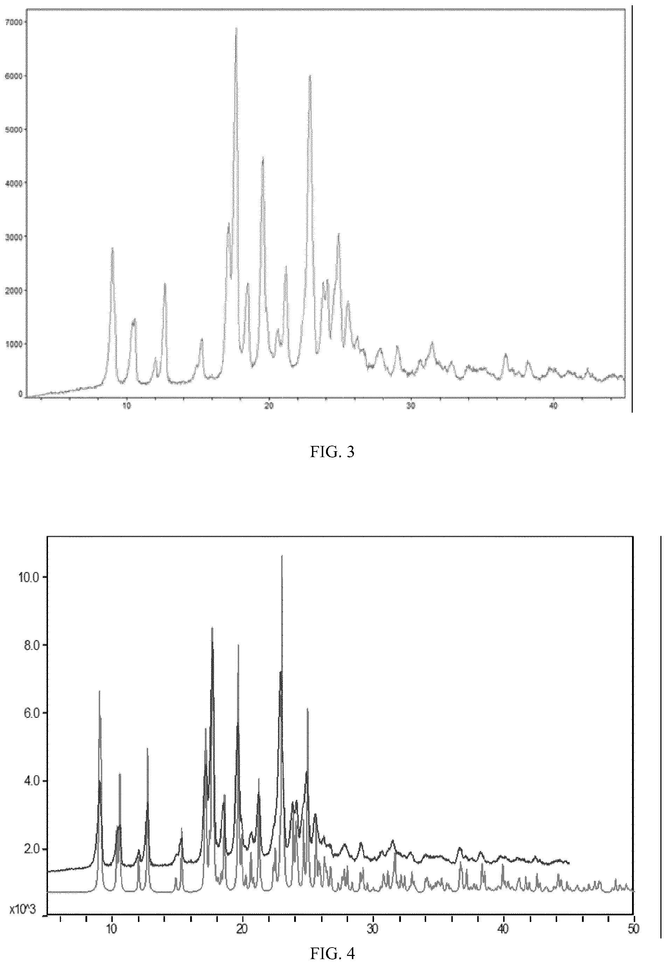

5 mg of the compound of formula (I) in free form was dissolved in 0.5 mL of ethyl acetate; 1.67 mg of *p*-toluenesulfonic acid was dissolved in 0.1 mL of tetrahydrofuran; the *p-*toluenesulfonic acid solution was slowly added to the compound of formula (I) solution with stirring, the mixture was stirred overnight and filtered, and the filter cake was dried in a vacuum drying oven at 40 °C, and subjected to DSC, TGA and XRPD analyses. The DSC and TGA graphs of the resulting *p*-toluenesulfonate salt of the compound of formula (I) are shown in FIG. 1 and FIG. 2, respectively, and the XRPD diffraction pattern is shown in FIG. 3.

### Example 2. Preparation of p-toluenesulfonate Salt of Compound of Formula (I)

2 g of the compound of formula (I) in free form was dissolved in 160 mL of ethyl acetate; 668 mg of *p*-toluenesulfonic acid was dissolved in 4 mL of tetrahydrofuran; the *p*-toluenesulfonic acid solution was slowly added to the compound of formula (I) solution with stirring, the mixture was stirred overnight and filtered, the filter cake was dried in a vacuum drying oven at 40 °C, and the XRPD diffraction pattern of the resulting *p*-toluenesulfonate salt of the compound of formula (I) is consistent with that in FIG. 3.

### Example 3. Preparation of p-toluenesulfonate Salt of Compound of Formula (I)

5 g of the compound of formula (I) in free form was dissolved/suspended in 400 mL of acetone; 1670 mg of *p*-toluenesulfonic acid was dissolved in 10 mL of tetrahydrofuran; the *p-*toluenesulfonic acid solution was slowly added to the compound of formula (I) solution with stirring, the mixture was stirred overnight and filtered, the filter cake was dried in a vacuum drying oven at 40 °C, and the XRPD diffraction pattern of the resulting *p*-toluenesulfonate salt of the compound of formula (I) is consistent with that in FIG. 3.

### Example 4. Preparation and Characterization of Single Crystal

150 mg of the *p*-toluenesulfonate salt of the compound of formula (I) was added to 1 mL of a vehicle (composition: methanol : ethanol (1:1), plus 2% water), the mixture was stirred at 50 °C for 15 min, the suspension was filtered through a 0.45 µm filter membrane, and the filtrate was used as a stock solution. 340 µL of the stock solution was well mixed with 260 µL of vehicle, and the mixture was cooled at room temperature and recrystallized to obtain the single crystal.

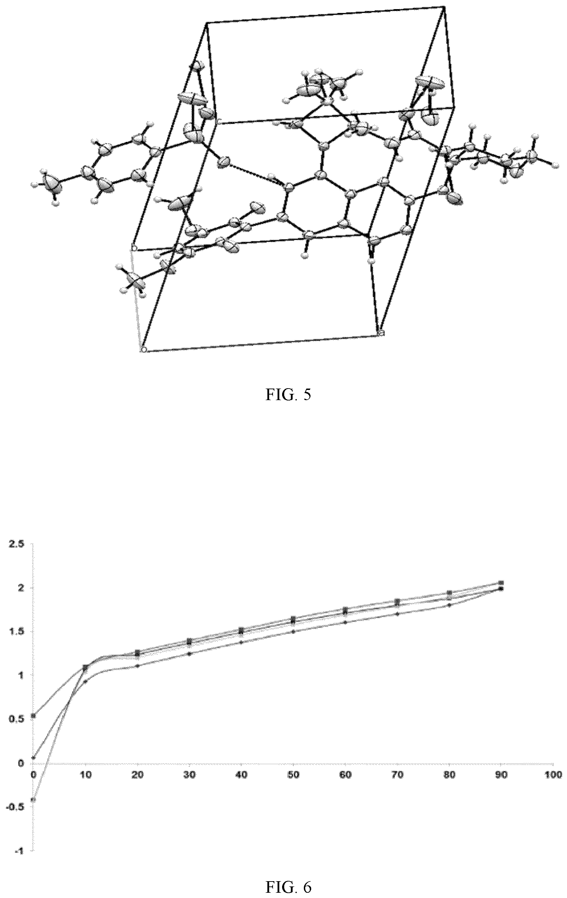

A proper single crystal was selected for analysis on a Bruker APEX-II CCD single-crystal diffractometer. The temperature was maintained at 220 K during data collection. The unit cell parameters of the *p*-toluenesulfonate salt of the compound of formula (I) are shown in Table 2; the single crystal XRPD simulation results are consistent with the XRPD results of the *p-*toluenesulfonate salt of the compound of formula (I), as shown in FIG. 4; the structure of the single crystal unit cell is shown in FIG. 5. Table 2:

| | |
|---|---|
| Molecular formula: C₃₅H₄₂F₂N₆O₉S | Z = 1 |
| Mᵣ = 760.80 | F(000) = 400.0 |
| Space group, P1 | Density Dₓ = 1.391 Mg m⁻³ |
| a = 9.079 (3) Å | Cu K radiation, = 1.54184 Å |
| b = 10.561 (3) Å | Linear absorption coefficient = 1.422 mm⁻¹ |
| c = 10.882 (7) Å | Test temperature T = 220 K |
| Unit cell volume V = 908.1 (4) Å³ | Size of single crystal: 0.15 * 0.08 * 0.03 mm |

### Example 5. Hygroscopic Behavior Test

The hygroscopic weight gains of the *p*-toluenesulfonate salt of the compound of formula (I) (Form A) in various relative humidity conditions (hygroscopic weight gain/pre-hygroscopic weight * 100%) were measured according to the dynamic vapor sorption method to assess the hygroscopicity of the *p*-toluenesulfonate salt of the compound of formula (I) (Form A). The results are shown in FIG. 6. Experimental results show that the weight change of the crystal of the *p*-toluenesulfonate salt of the compound of formula (I) (Form A) of the present invention is generally no more than 2% along with the increase of relative humidity (%), and the *p-*toluenesulfonate salt of the compound maintains relatively good hygroscopicity, meets the requirements for industrial production, and therefore can satisfy the needs of clinical drug formulation development.

### Example 6. Stability Test

The p-toluenesulfonate salt of the compound of formula (I) (Form A) and the compound of formula (I) in free form were subjected to conditions of high temperature and accelerated experiments for stability test, and the chemical purity was used as an evaluation index to evaluate the stability of the *p*-toluenesulfonate salt of the compound of formula (I) (Form A) and the compound of formula (I) in free form. The experimental results are shown in Table 3:

| Compound | Chemical stability (% initial) | | | | | |
|---|---|---|---|---|---|---|
| | 60°C | | | 40°C/75%RH | | |
| | 3d | 7d | 14d | 3d | 7d | 14d |
| *p*-toluenesulfonate salt of the compound of formula (I) (Form A) | 100.3 | 99.8 | 99.8 | 100.2 | 100.1 | 100.0 |
| Compound of formula (I) in free form | 100.1 | 100.1 | 100.1 | 99.9 | 100.0 | 100.0 |

The above experimental results show that the compound of the present invention can remain stable within 14 days under the conditions of high temperature and accelerated experiments, the chemical stability of the product meets the requirements for industrial production, and the compound can satisfy the needs of clinical drug formulation development.

### Example 7. Solubility Determination

### 1) Solubility in water

2 mg of the *p*-toluenesulfonate salt of the compound of formula (I) (Form A) and 2 mg of the compound of formula (I) in free form were separately placed in a 2 mL glass bottle, 0.4 mL of water was added into the glass bottles separately, the two mixtures were each stirred for 24 hrs on a magnetic stirrer at room temperature and then centrifuged, and the content measurement was carried out by using a liquid phase. The experimental results are shown in Table 4:

| Compound | Vehicle | Solubility (µg/mL) |
|---|---|---|
| *p*-toluenesulfonate salt of the compound of formula (I) (Form A) | Water | 1370 |
| Compound of formula (I) in free form | Water | 25.6 |

It can be seen from the above experimental results that the solubility of the *p-*toluenesulfonate salt of the compound of formula (I) (Form A) in water is significantly higher than that of the compound of formula (I) in free form, and the *p*-toluenesulfonate salt of the compound of formula (I) (Form A) can satisfy the needs of clinical drug formulation development.

### 2) Solubility in physiological vehicle

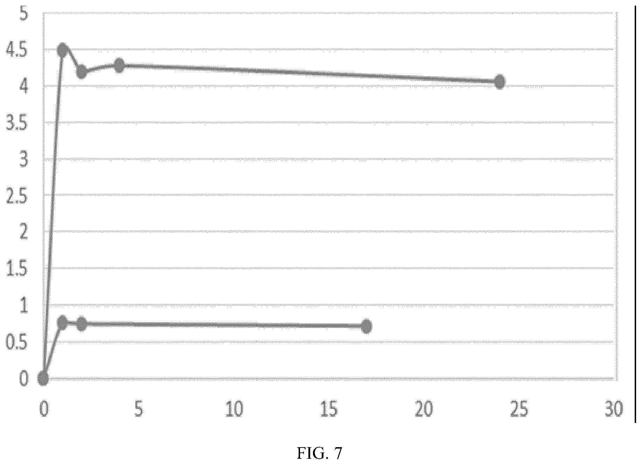

80 mg of the *p*-toluenesulfonate salt of the compound of formula (I) (Form A) and 80 mg of the compound of formula (I) in free form were separately placed in a 4 mL glass bottle, 2 mL of vehicle (25% PEG300 / 5% Solutol HS15 / 70% water) (v/v/v) was added into the glass bottles separately, the two mixtures were each stirred on a magnetic stirrer at room temperature, samples were taken at various time points and centrifuged, and the content measurement was carried out by using a liquid phase. The experimental results are shown in FIG. 7. It can be seen from the experimental results that the solubility of the *p*-toluenesulfonate salt of the compound of formula (I) is significantly higher than that of the compound of formula (I) in free form, and the *p-*toluenesulfonate salt of the compound of formula (I) can satisfy the needs of clinical drug formulation development.

### Example 8. In Vivo Pharmacokinetic Experiment in Animals

The *p*-toluenesulfonate salt of the compound of formula (I) (Form A) and the compound of formula (I) in free form were each subjected to PK experiments in monkeys, and the administration dose was 200 mpk (based on the compound in free form). The results are shown in Table 5:

| Pharmaceutical compound | Animal species | Administration dose | AUC(hr*ng/mL) |
|---|---|---|---|
| *p*-toluenesulfonate salt of the compound of formula (I) (Form A) | Monkey | 200 mpk | 124860 |
| Compound of formula (I) in free form | Monkey | 200 mpk | 9952 |

The above experimental results show that the *p*-toluenesulfonate salt of the compound of formula (I) can significantly improve the exposure of the compound of formula (I) in free form in monkeys, and therefore it can be seen that the bioavailability of the *p*-toluenesulfonate salt of the compound of formula (I) (Form A) is significantly higher than that of the compound of formula (I) in free form.

All documents mentioned in the present invention are incorporated as references, just as each document is individually cited as a reference. In addition, it should be understood that various modifications or changes may be made by those skilled in the art after reading the content of the present invention, and these equivalent forms also fall within the scope defined by the claims appended hereto.

## Claims

1. A *p*-toluenesulfonate salt of a compound of formula (I):

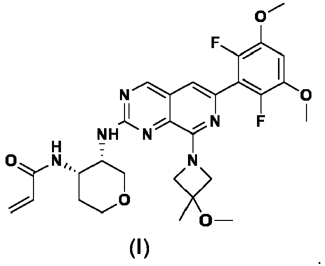

2. The *p*-toluenesulfonate salt of the compound of formula (I) of claim 1, wherein the *p-*toluenesulfonate salt of the compound of formula (I) is a crystalline compound.

3. The *p*-toluenesulfonate salt of the compound of formula (I) of claim 2, wherein an X-ray powder diffraction pattern (XRPD) of the *p*-toluenesulfonate salt of the compound of formula (I) comprises peaks at angles of diffraction (2Θ) of 9.02 ± 0.2°, 17.18 ± 0.2°, 17.68 ± 0.2°, 19.60 ± 0.2° and 22.90 ± 0.2°; **preferably,** an X-ray powder diffraction pattern (XRPD) of the *p-*toluenesulfonate salt of the compound of formula (I) further comprises peaks at angles of diffraction (2Θ) of 10.60 ± 0.2°, 12.72 ± 0.2°, 18.56 ± 0.2°, 21.22 ± 0.2° and 24.90 ± 0.2°; **more preferably,** an X-ray powder diffraction pattern (XRPD) of the *p*-toluenesulfonate salt of the compound of formula (I) further comprises peaks at angles of diffraction (2Θ) of 15.32 ± 0.2°, 23.82 ± 0.2°, 24.12 ± 0.2°, 24.60 ± 0.2° and 25.58 ± 0.2°.

4. The *p*-toluenesulfonate salt of the compound of formula (I) of claim 2, wherein an X-ray powder diffraction pattern of the *p*-toluenesulfonate salt of the compound of formula (I) comprises peaks at angles of diffraction (2Θ) of 9.02 ± 0.2°, 10.60 ± 0.2°, 12.04 ± 0.2°, 12.72 ± 0.2°, 15.32 ± 0.2°, 17.18 ± 0.2°, 17.68 ± 0.2°, 18.56 ± 0.2°, 19.60 ± 0.2°, 20.60 ± 0.2°, 21.22 ± 0.2°, 22.90 ± 0.2°, 23.82 ± 0.2°, 24.12 ± 0.2°, 24.60 ± 0.2°, 24.90 ± 0.2°, 25.58 ± 0.2°, 27.78 ± 0.2°, 29.04 ± 0.2°, 31.48 ± 0.2°, 36.60 ± 0.2° and 38.16 ± 0.2°.

5. The *p*-toluenesulfonate salt of the compound of formula (I) of any one of claims 1-4, wherein the unit cell of the *p*-toluenesulfonate salt of the compound of formula (I) is P1, a = 9.079 (3) Å, b = 10.561 (3) Å, c = 10.882 (3) Å, and the unit cell volume is 908.1 (4) Å³.

6. The *p*-toluenesulfonate salt of the compound of formula (I) of any one of claims 1-4, wherein the *p*-toluenesulfonate salt of the compound of formula (I) is a hydrate; **preferably,** the hydrate comprises 1-3 water molecules per molecule; **more preferably,** the hydrate comprises 1, 2 or 3 water molecules per molecule.

7. A preparation method for the *p*-toluenesulfonate salt of the compound of formula (I) of claim 1, comprising the following steps:
1) dissolving or dispersing the compound of formula (I) in free form in an aqueous solvent or a suitable organic solvent, and adding *p*-toluenesulfonic acid or a *p*-toluenesulfonic acid solution to the above system for a salt forming reaction; or adding the compound of formula (I) in free form to a *p*-toluenesulfonic acid solution for a salt forming reaction;
2) collecting a *p*-toluenesulfonate salt of the compound of formula (I) in the form of a solid product precipitated during the salt forming reaction; or creating a degree of supersaturation of the salt forming system to obtain a *p*-toluenesulfonate salt of the compound of formula (I) in the form of a solid product.

8. The preparation method of claim 7, wherein a method for creating the degree of supersaturation of the salt forming system in step 2) is one or more of: adding a seed crystal, volatilizing a solvent, adding an anti-solvent and cooling.

9. The method of claim 7, wherein the suitable organic solvent used in step 1) is selected from the group consisting of alcohols, chloroalkanes, ketones, ethers, cyclic ethers, esters, alkanes, cycloalkanes, benzenes, amides, sulfoxides and mixtures thereof.

10. The method of claim 9, wherein the suitable organic solvent is selected from the group consisting of methanol, ethanol, *n*-propanol, isopropanol, dichloromethane, acetonitrile, acetone, 1,4-dioxane, tetrahydrofuran, *N,N*-dimethylformamide, ethyl acetate, isopropyl acetate, methyl *tert*-butyl ether, 2-methoxyethyl ether and mixtures thereof.

11. A pharmaceutical composition, comprising a therapeutically effective dose of the *p-*toluenesulfonate salt of the compound of formula (I) of any one of claims 1-6 and a pharmaceutically acceptable carrier.

12. Use of the *p*-toluenesulfonate salt of the compound of formula (I) of any one of claims 1-6 in the preparation of a medicament as an FGFR4 inhibitor.

13. Use of the *p*-toluenesulfonate salt of the compound of formula (I) of any one of claims 1-6 in the preparation of a medicament for treating liver cancer, prostate cancer, pancreatic cancer, esophageal cancer, gastric cancer, lung cancer, breast cancer, ovarian cancer, colon cancer, skin cancer, glioblastoma or rhabdomyosarcoma.

14. A method for treating liver cancer, prostate cancer, pancreatic cancer, esophageal cancer, gastric cancer, lung cancer, breast cancer, ovarian cancer, colon cancer, skin cancer, glioblastoma or rhabdomyosarcoma, comprising administering to a patient in need thereof a therapeutically effective amount of the *p*-toluenesulfonate salt of the compound of formula (I) of any one of claims 1-6 or the pharmaceutical composition of claim 11.
